(19) Europäisches Patentamt
European Patent Office
Office européen des brevets

(11) Numéro de publication: **0 030 174**
**A1**

(12) # DEMANDE DE BREVET EUROPEEN

(21) Numéro de dépôt: **80401586.5**

(22) Date de dépôt: **06.11.80**

(51) Int. Cl.³: **C 07 D 401/04**
C 07 D 401/14, A 61 K 31/445

(30) Priorité: **15.11.79 FR 7928162**

(43) Date de publication de la demande:
**10.06.81 Bulletin 81/23**

(84) Etats Contractants Désignés:
**AT BE CH DE GB IT LI NL SE**

(71) Demandeur: **SCIENCE UNION ET Cie SOCIETE FRANCAISE DE RECHERCHE MEDICALE**
**14 rue du Val d'Or**
**F-92150 Suresnes(FR)**

(72) Inventeur: **Regnier, Gilbert**
**Av. du Plessis**
**F-92290 Chatenay-Malabry(FR)**

(72) Inventeur: **Duhault, Jaques**
**14 bis rue Paul Demange**
**F-78290 Croissy/Seine(FR)**

(72) Inventeur: **Boulanger, Michelle**
**71 av. Auguste Renoir**
**F-78160 Marly-le-Roi(FR)**

(72) Inventeur: **Dhainaut, Alain**
**16 rue Maurice Ravel**
**F-92230 Gennevilliers(FR)**

(74) Mandataire: **Reverbori, Marcelle**
**SCIENCE UNION ET Cie SOCIETE FRANCAISE DE RECHERCHE MEDICALE Service Brevets 22 rue Garnier**
**F-92200 Neuilly/Seine(FR)**

(54) **Nouveaux dérivés de la pipéridylbenzimidazolinone, leurs procédés de préparation et les compositions pharmaceutiques les renfermant.**

(57) Nouveaux dérivés de la pipéridylbenzimidazolinone utilisables comme médicament et répondant à la formule:

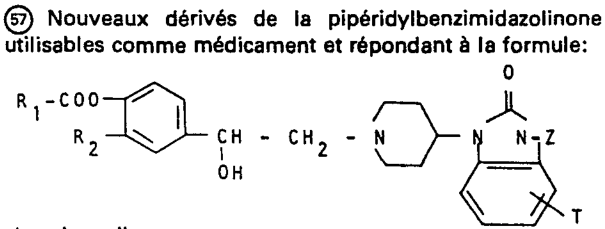

dans laquelle:

$R_1$ est alkyle jusqu'en $C_6$, phényle, halogénophényle, alkylphényle, alkoxyphényle, méthylènedioxyphényle, éthylènedioxyphényle, thiényle, furyle ou pyridyle,
$R_2$ est $R'_1COO$- ou $R'_1$-COO-$CH_2$- dans lesquels $R'_1$ a les mêmes significations que $R_1$, $R_1$ et $R'_1$ étant identiques ou différents, ou $R_3NH$- dans lequel $R_3$ est formyle, acétyle, mésyle, carbamoyle, sulfamoyle ou éthoxalyle,
Z est hydrogène, alkyle ou alcényle jusqu'en $C_6$ et
T est hydrogène, halogène, alkyle ou alcoxy jusqu'en $C_6$.

Ces nouveaux composés et leurs sels physiologiquement tolérables peuvent être utilisés en thérapeutique notamment dans le traitement des maladies autoimmunes, allergiques et inflammatoires et dans le traitement des dyspnées notamment asthmatique et bronchique.

EP 0 030 174 A1

0030174

Nouveaux dérivés de la pipéridylbenzimidazolinone, leurs procédés de préparation et les compositions pharmaceutiques les renfermant.

La présente invention a pour objet des dérivés de la pipéridylbenzimidazolinone, leurs procédés de préparation et les compositions pharmaceutiques les renfermant.

Elle concerne particulièrement les dérivés de la pipéridylbenzimidazolinone de formule générale I :

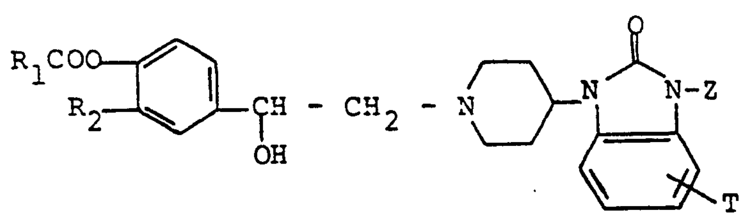

(I)

dans laquelle :

$R_1$ représente :

un radical alkyle ayant de 1 à 6 atomes de carbone, en chaîne droite ou ramifiée,

un radical phényle non substitué ou substitué par un ou plusieurs substituants choisis parmi les atomes d'halogène, et les radicaux alkyle et alcoxy ayant chacun de 1 à 5 atomes de carbone, méthylènedioxy ou éthylènedioxy, ou

un radical thiényle, furyle ou pyridile ;

$R_2$ représente :

un radical $R'_1$-COO- ou $R'_1$-COO-$CH_2$- dans lesquels $R'_1$ a les mêmes significations que $R_1$, $R_1$ et $R'_1$ pouvant être identiques ou différents, ou
un radical $R_3NH$- dans lequel $R_3$ représente un radical formyle, acétyle, mésyle, carbamoyle, sulfamoyle ou éthoxalyle ;
Z représente un atome d'hydrogène ou un radical alkyle ou alcényle renfermant chacun jusqu'à 5 atomes de carbone en chaîne droite ou ramifiée, et
T représente un atome d'hydrogène ou d'halogène ou un radical alkyle ou alcoxy ayant chacun de 1 à 5 atomes de carbone en chaîne droite ou ramifiée.

La présente invention a également pour objet le procédé de préparation desdérivés de formule générale I caractérisé en ce que l'on réduit un composé de formule générale II :

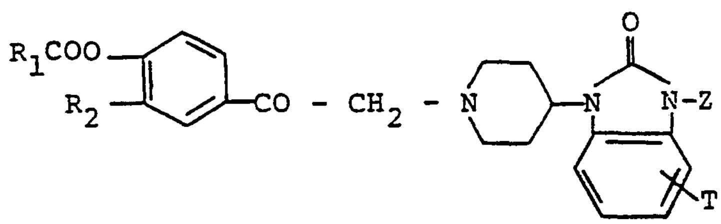

(II)

dans laquelle $R_1$, $R_2$, Z et T ont les significations définies précédemment.

La réduction du composé II est accomplie de manière particulièrement adéquate au moyen d'un hydrure alcalin tel que le borohydrure de sodium ($NaBH_4$) ou de potassium ($KBH_4$) ou du cyanoborohydrure de sodium ($NaBH_3CN$), dans un solvant tel que le méthanol, l'éthanol ou le tétrahydrofuranne, en milieu anhydre ou aqueux, à un pH compris entre 8 et 4 selon la nature de l'agent réducteur. Ctte réduction peut également être avantageusement réalisée par l'hydrogène sous une pression comprise entre 5 et 60 bars, en présence de métaux du 8ème groupe comme catalyseurs tels que le palladium sur charbon,

ou le platine, dans un solvant polaire tel qu'un alcool à bas point d'ébullition miscible à l'eau, à une température comprise entre 25 et 80°C.

Les composés de formule générale II ont été préparés par estérification des composés de formule IIa :

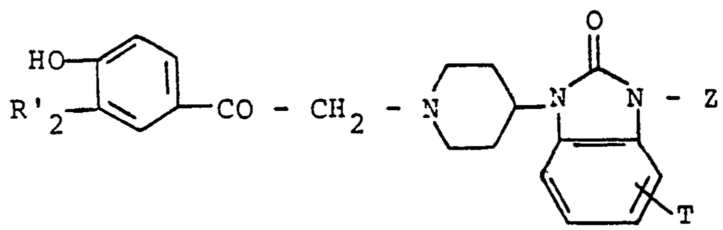

(IIa)

dans laquelle Z et T ont les significations énoncées précédemment et $R'_2$ représente un radical hydroxyle, hydroxyméthyle ou $R_3NH-$ dans lequel $R_3$ a la signification définie précédemment, au moyen d'un chlorure d'acide de formule : $R_1-CO-Cl$ dans laquelle $R_1$ a la signification précédemment définie, selon des techniques analogues à celles décrites par B. TULLAR et coll. J. med. chem. 19, 834 (1976)

Les composés de formule $II_a$ ont eux-mêmes été préparés en faisant réagir une halo-acétophénone de formule générale IIb :

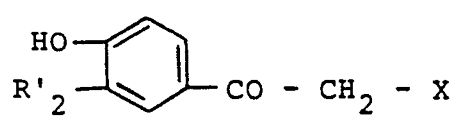

(IIb)

dans laquelle $R'_2$ a la signification précédemment définie et X représente un atome de chlore ou de brome, sur une pipéridylbenzimidazolinone de formule générale III :

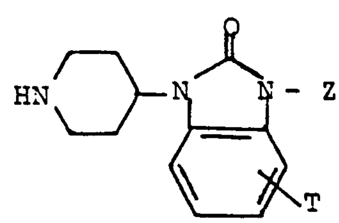

(III)

dans laquelle Z et T ont les significations énoncées précédemment. Il est avantageux d'effectuer une telle réaction dans un solvant polaire tel qu'un alcool ou une cétone aliphatique ayant 4 ou 5 atomes de carbone ou le diméthylformamide, à une température comprise entre 110 et 140°C, en présence d'un accepteur de l'hydracide formé au cours de la réaction. Comme accepteurs, on peut utiliser par exemple les carbonates alcalins tels que les carbonates de sodium ou de potassium ou un excès de la pipéridylbenzimidazolinone III.

Les haloacétophénones de formule IIb sont soit connues et décrites dans la littérature, soit préparées selon la méthode décrite par LARSEN et coll. J. Med. Chem. 10, 462 (1967).

Les (pipéridyl-4)-1 benzimidazolinones-2 de formule III sont préparées à partir des (triphénylméthyl-1 pipéridyl-4)-1 benzimidazolinones-2 correspondantes.

La (pipéridyl-4)-1 benzimidazolinone-2 est un produit du commerce.

La présente invention a également pour objet le procédé de préparation des composés de formule générale I caractérisé en ce que l'on effectue une réduction alcoylante du mélange de la pipéridylbenzimidazolinone de formule générale III précédemment définie et d'un phénylglyoxal de formule générale IV :

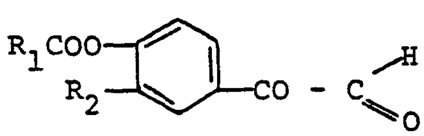
(IV)

dans laquelle $R_1$ et $R_2$ ont les significations précédemment définies.

Il est particulièrement avantageux d'effectuer

cette réduction alcoylante, selon une méthode analogue à celle décrite par G. FODOR et coll., Am. Soc. 71, 1045 (1949), en opérant sous une pression d'hydrogène comprise entre 5 et 10 bars en présence de catalyseurs choisis parmi les métaux du 8ème groupe, tel que par exemple le platine ou le palladium sur charbon, dans un solvant polaire tel qu'un alcool aliphatique miscible à l'eau, à une température comprise entre 25 et 80°C.

Les phénylglyoxals de formule générale IV sont préparés selon la technique de G. FODOR et coll. Am. Soc. 71, 1045 (1949).

La présente invention a aussi pour objet le procédé de préparation des dérivés de formule générale I caractérisé en ce que l'on fait réagir la pipéridylbenzimidazolinone de formule III précédemment définie avec une halohydrine de formule générale V :

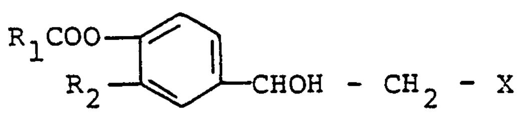

(V)

dans laquelle $R_1$ et $R_2$ sont tels que précédemment définis, et X représente un atome de chlore ou de brome.

Une telle réaction est effectuée avantageusement dans un solvant tel que le diméthylformamide ou le diglyme à une température comprise entre 110 et 140°C, en pr:sence d'un accepteur de l'hydracide formé au cours de la réaction. Comme accepteurs on peut employer par exemple la triéthylamine ou un excès de la pipéridylbenzimidazolinone de formule III.

Les halohydrines de départ de formule générale V sont communément préparées par exemple à partir des haloacétophénones correspondantes par réduction au moyen d'un borohydrure alcalin. Elles sont le plus souvent utilisées à l'état brut sans purification.

Les composés de formule générale I obtenus selon les procédés ci-dessus sont des bases faibles. La présence de fonctions esters phénoliques labiles rend leur purification particulièrement délicate. Ils peuvent donc être purifiés soit par chromatographie sur alumine neutre, soit par cristallisation, à l'état de base ou de sel, dans des solvants polaires tels que les alcools aliphatiques, l'acétonitrile ou le tétrahydrofuranne.

La présente invention inclut également les sels des dérivés de formule générale I avec des acides minéraux et organiques compatibles, et plus particulièrement ceux qui sont physiologiquement tolérables. Parmi les acides utilisables pour la formation de ces sels on peut citer par exemple les acides chlorohydrique, bromhydrique, sulfurique, méthanesulfonique et iséthionique.

Les dérivés de formule générale I et leurs sels physiologiquement tolérables possèdent des propriétés pharmacologiques et thérapeutiques intéressantes, notamment des propriétés bronchodilatatrice, β-adrénergique et antiallergique.

Leur toxicité est faible et leur $DL_{50}$ déterminée chez la souris est supérieure à 100mg/kg par voie intrapéritonéale, et supérieure à 800mg/kg par voie orale.

L'activité bronchodilatatrice a été étudiée chez le cobaye par la méthode de H. KONZETT et R. ROSSLER, Arch. Exp. Path. U. Pharm. 195, 71 (1940). On a ainsi observé que les dérivés de la présente invention injectés par voie intraveineuse à des doses variant, selon les dérivés, de 0,05 à 1mg/kg, inhibent totalement le bronchospasme provoqué par l'administration intraveineuse soit d'histamine, soit de sérotonine, soit d'acétylcholine, et l'effet de la Slow Reacting Substance, à la dose de 0,100 à 0,250mg/kg.

Soumis au test de A.K. Armitage, Brit. J. Pharmacol. 17, 196, (1961), les dérivés de l'invention administrés par voie intrapéritonéale à des doses variant de 0,5 à 2,5mg/kg selon les composés, inhibent de 50% l'effet produit chez le cobaye par un aérosol d'histamine à 4%.

Les dérivés de l'invention, administrés par voie intrapéritonéale, à des doses de 2,5 à 5mg/kg selon les composés, inhibent de 50% le choc anaphylactique provoqué par l'administration d'un aérosol d'albumine à 5% à des cobayes préalablement sensibilisés à l'albumine. Ce test est mis en oeuvre de la façon suivante, sur des lots de 6 cobayes par produit à tester.

Les cobayes sont soumis à une injection intrapéritonéale de 100mg/kg d'ovalbumine en émulsion dans l'adjuvant de Freund. Quatre semaines plus tard, on procède à une sélection des cobayes sensibilisés. Pour celà, les animaux à jeun depuis la veille sont soumis à un aérosol d'ovalbumine à 5% et les temps d'apparition de dyspnée sévère puis de pré-coma sont notés. On sélectionne alors pour les tests suivants, les cobayes, chez lesquels une dyspnée sévère apparaît moins de 3 minutes après le début du traitement à l'aérosol d'ovalbumine. Huit jours plus tard, sur ces cobayes sélectionnés maintenus à jeun depuis la veille, on injecte par voie intrapéritonéale les produits à tester, puis 20 minutes plus tard on soumet alors ces cobayes à un aérosol d'ovalbumine à 5%. On note les temps d'apparition de dyspnée sévère puis de pré-coma, et on détermine ainsi le rôle protecteur des produits testés.

D'autre part, on a observé une action sur l'anaphylaxie cutanée passive provoquée chez le rat selon la technique d'Ovary, Prog. Allergy 5, 459-508, S. Karger Basel / New York, avec les dérivés de l'invention administrés par voie intraveineuse, à des doses variant de 0,250 à 2,5mg/kg selon les composés.

Les propriétés pharmacologiques ci-dessus décrites, ainsi que la faible toxicité des dérivés de formule générale I et de leurs sels physiologiquement tolérables permettent leur utilisation en thérapeutique notamment dans le traitement de toutes les maladies où il est nécessaire d'inhiber les réactions antigène-anticorps comme les maladies autoimmunes, allergiques et inflammatoires et en particulier celles dans lesquelles un effet β-adrénergique additionnel est bienvenu telles la dyspnée asthmatique, et celle de la bronchite chronique.

La présente invention a également pour objet les compositions pharmaceutiques contenant comme principe actif un dérivé de formule générale I, ou un de ses sels physiologiquement tolérables, mélangé ou associé à un excipient pharmaceutique approprié, et notamment à un véhicule convenant à une administration par aérosol.

Les compositions pharmaceutiques ainsi obtenues sont présentées avantageusement sous des formes dosées diverses, telles que par exemple comprimés, dragées, gélules, glossettes ou préparations galéniques appropriées pour une administration sublinguale, suppositoires, solutions injectables ou buvables, ainsi que sous des formes adaptées à l'administration par aérosol. A titre indicatif, pour le dérivé de l'exemple 1 décrit ci-après, on peut utiliser lors d'administration par aérosol des doses de 250 à 1000μg de principe actif par bouffée, et lors d'administration orale des doses de 20 à 80mg, une à trois fois par jour.

Les exemples suivants illustrent l'invention, les points de fusion étant, sauf mention contraire, déterminés au tube capillaire.

EXEMPLE 1 :

[(bis para-toluoyloxy-3,4 phényl)-2 hydroxy-2]éthyl-1 (oxo-2 benzimidazolinyl-1)-4 pipéridine :

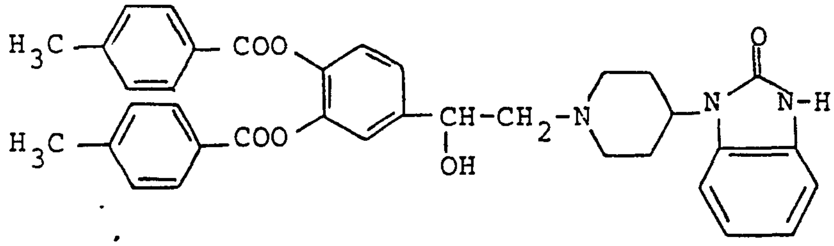

Première méthode :

On ajoute en une heure, sous agitation, un mélange de 0,38g de borohydrure de sodium et 15ml d'eau à une solution de 6g (0,01 mole) de [(bis para-toluoyloxy-3,4 phényl)-2 oxo-2] éthyl-1 (oxo-2 benzimidazolinyl-1)-4 pipéridine dans 200ml de tétrahydrofuranne, en maintenant la température à 5°C. Le milieu réactionnel est ensuite maintenu sous agitation pendant 1 heure 1/2 à la température de 5°C, puis il est concentré sous vide. Le résidu est repris par 150ml de chloroforme, lavé avec 80ml d'une solution aqueuse à 5% de bicarbonate de sodium, puis 3 fois avec 150ml d'eau. La phase organique est séchée sur sulfate de magnésium, puis concentrée sous vide. L'huile résiduelle, dissoute dans 10ml d'éthanol acidifié par une solution de gaz chlorhydrique dans l'éther, cristallise pour donner 2g de chlorhydrate de [(bis para-toluoyloxy-3,4 phényl)-2 hydroxy-2] éthyl-1 (oxo-2 benzimidazolinyl-1)-4 pipéridine, P.F. 220-226°C. (Rendement 31%)

Le [(bis para-toluoyloxy-3,4 phényl)-2 oxo-2] éthyl-1 (oxo-2 benzimidazolinyl-1)-4 pipéridine de départ, P.F. 168-172°C cristallisé dansl'acétonitrile, a été obtenu par addition de 0,16 mole de chlorure de para-toluoyle dans une solutionde 600ml de dioxanne contenant 0,16 mole de triéthylamine et 0,04 mole d'[(oxo-2 benzimidazolinyl-1)-4 pipéridyl-1]-2 dihydroxy-3',4' acétophénone, elle-même

préparée à partir de dihydroxy-3,4 phényl chlorométhylcétone (produit commercial) et d'(oxo-2 benzimidazolinyl-1)-4 pipéridine (produit commercial).

Deuxième méthode :

Une solution de 20,15g de di para-toluoyloxy-3,4 phényl-glyoxal, P.F. de la dinitrophénylhydrazone correspondante : 262°C, (préparé selon la technique de G. FODOR et coll. Am. Soc. 71, 1045 (1949) à partir de la di paratoluoyloxy-3,4 acétophénone fondant à 155-157°C) et de 10,85g d'(oxo-2 benzimidazolinyl-1)-4 pipéridine dans 500ml d'éthanol à 90% est soumise à l'hydrogénation sous 10 bars en présence de 7,5g de charbon palladié renfermant 10% en poids de palladium. Lorsque la quantité théorique d'hydrogène est absorbée, on filtre le catalyseur et évapore le solvant sous pression réduite. Le résidu cristallin est agité avec 2 fois 100ml d'eau bouillante en présence de chloroforme. L'extrait chloroformique est décanté et le solvant évaporé sous pression réduite pour donner 15g d'une base huileuse brute. Après dissolution de cette base dans 150ml d'éthanol et addition d'une solution de gaz chlorhydrique dans l'éther jusqu'à pH7, on isole 14,2g de chlorhydrate de [(bis para-toluoyloxy-3,4 phényl)-2 hydroxy-2] éthyl-1 (oxo-2 benzimidazolinyl-1)-4 pipéridine, P.F. 223-226°C.

Troisième méthode :

On chauffe pendant 8 heures à reflux une solution de 10,6g de (di para-toluoyloxy-3,4 phényl)-1 chloro-2 éthanol-1 (huile, préparé à partir de di para-toluoyloxy-3,4 phényl chlorométhylcétone, P.F. 113°C, selon une méthode analogue à celle de O. HINSBERG, brevet allemand N°364.039 du 16 novembre 1922) et de 10,8g d'(oxo-2 benzimidazolinyl-1)-4 pipéridine dans 250ml de diglyme. Lorsque la réaction est terminée, on filtre et évapore le solvant sous pression réduite. Le résidu est agité avec 2 fois 50ml d'eau bouillante

en présence de chloroforme. L'extrait chloroformique est décanté et le solvant évaporé sous pression réduite. Le résidu huileux obtenu est dissout dans 150ml d'éthanol et additionné d'une solution de gaz chlorhydrique dans l'éther jusqu'à pH7. On isole finalement 8,2g de chlorhydrate de [(bis para-toluoyloxy-3,4 phényl)-2 hydroxy-2] éthyl-1 (oxo-2 benzimidazolinyl-1)-4 pipéridine, P.F. 220-225°C.

EXEMPLE 2 :

[(bis triméthylacétoxy-3,4 phényl)-2 hydroxy-2] éthyl-1 (oxo-2 benzimidazolinyl-1)-4 pipéridine

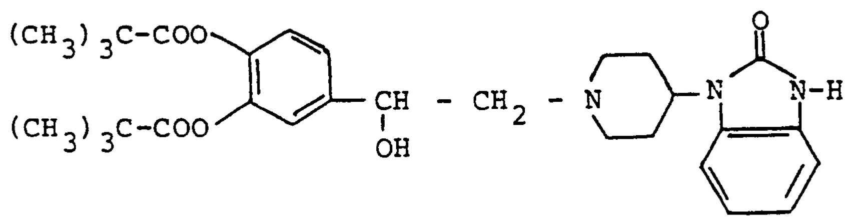

5,72g (0,01 mole) de chlorhydrate de [(bis triméthylacétoxy-3,4 phényl)-2 oxo-2] éthyl-1 (oxo-2 benzimidazolinyl-1)-4 pipéridine en solution dans120ml de méthanol anhydre sont hydrogénés pendant 16 heures à 60°C sous une pression de 50 bars, en présence de 1g de charbon palladié renfermant 10% de palladium. La solution est ensuite filtrée à chaud sur célite en présence de charbon activé. Le filtre est rincé avec 2 fois 50ml de méthanol chaud. La solution alcoolique est concentrée sous vide. L'huile résiduelle cristallise dans un mélange de 8ml d'éthanol et 10ml d'éther de pétrole pour donner 2,8g de chlorhydrate de [(bis triméthylacétoxy-3,4 phényl)-2 hydroxy-2] éthyl-1 (oxo-2 benzimidazolinyl-1)-4 pipéridine, P.F. 168°C, (Rendement 50%).

Le chlorhydrate de [(bis triméthylacétoxy-3,4 phényl)-2oxo-2] éthyl-1 (oxo-2 benzimidazolinyl-1)-4 pipéridine de départ, P.F. 260-268°C (isopropanol), a été obtenu par addition de 0,16 mole de chlorure de pivaloyle à une

solution de 600ml de dioxanne, 0,16 mole de triéthylamine et 0,04 mole d'[(oxo-2 benzimidazolinyl-1)-4 pipéridyl-1] -2 dihidroxy-3',4' acétophénone. Ce dernier composé, dont le chlorhydrate fond à 307-313°C, a lui-même été obtenu par condensation d'ω-chloro dihydroxy-3,4 acétophénone avec l'(oxo-2 benzimidazolinyl-1)-4 pipéridine, en présence de carbonate de potassium dans la méthyléthylcétone.

Le [(bis triméthylacétoxy-3,4 phényl)-2 hydroxy-2] éthyl-1 (oxo-2 benzimidazolinyl-1)-4 pipéridine, a également été préparé selon les méthodes décrites dans l'exemple 1, à savoir :

par réduction au moyen de borohydrure de sodium de [(bis triméthylacétoxy-3,4 phényl)-2 oxo-2] éthyl-1 (oxo-2 benzimidazolinyl-1)-4 pipéridine,

par réduction alcoylante du mélange de bis triméthylacétoxy-3,4 phénylglyoxal et d'(oxo-2 benzimidazolinyl-1)-4 pipéridine, et,

en faisant réagir le (bis triméthylacétoxy-3,4 phényl)-1 chloro-2 éthanol-1 avec l'(oxo-2 benzimidazolinyl-1)-4 pipéridine.

EXEMPLES 3 à 14 :

Les composés suivants ont été préparés selon les méthodes décrites dans les exemples 1 et 2 :

3) [(bis para-anisoyloxy-3,4 phényl)-2 hydroxy-2] éthyl-1 (oxo-2 benzimidazolinyl-1)-4 pipéridine, P.F. 137-143°C (cyanure de méthyle) à partir :

de [(bis para-anisoyloxy-3,4 phényl)-2 oxo-2] éthyl-1 (oxo-2 benzimidazolinyl-1)-4 pipéridine, ou

de bis para-anisoyloxy-3,4 phénylglyoxal et d'(oxo-2 benzimidazolinyl-1)-4 pipéridine, ou

de (bis para-anisoyloxy-3,4 phényl)-1 chloro-2 éthanol-1 et d'(oxo-2 benzimidazolinyl-1)-4 pipéridine.

4) [(bis benzoyloxy-3,4 phényl)-2 hydroxy-2] éthyl-1 (oxo-2

benzimidazolinyl-1)-4 pipéridine, P.F. du chlorhydrate correspondant : 156°C (éthanol), à partir :

de [(bis benzoyloxy-3,4 phényl)-2 oxo-2] éthyl-1 (oxo-2 benzimidazolinyl-1)-4 pipéridine, P.F. 159-163°C, ou de bis benzoyloxy-3,4 phénylglyoxal et d'(oxo-2 benzimidazolinyl-1)-4 pipéridine, ou

de (bis benzoyloxy-3,4 phényl)-1 chloro-2 éthanol-1 et d'(oxo-2 benzimidazolinyl-1)-4 pipéridine.

5) [(bis ortho-toluoyloxy-3,4 phényl)-2 hydroxy-2] éthyl-1 (oxo-2 benzimidazolinyl-1)-4 pipéridine, P.F. du chlorhydrate correspondant : 165-172°C (éthanol) à partir :

de [(bis ortho-toluoyloxy-3,4 phényl)-2 oxo-2] éthyl-1 (oxo-2 benzimidazolinyl-1)-4 pipéridine, P.F. du chlorhydrate correspondant : 224-229°C, ou

de bis ortho-toluoyloxy-3,4 phénylglyoxal et d'(oxo-2 benzimidazolinyl-1)-4 pipéridine, ou

de (bis ortho-toluoyloxy-3,4 phényl)-1 chloro-2 éthanol-1 et d'(oxo-2 benzimidazolinyl-1)-4 pipéridine.

6) [(bis méta-toluoyloxy-3,4 phényl)-2 hydroxy-2] éthyl-1 (oxo-2 benzimidazolinyl-1)-4 pipéridine, P.F. du chlorhydrate correspondant : 186°C (éthanol), à partir :

de [(bis méta-toluoyloxy-3,4 phényl)-2 oxo-2] éthyl-1 (oxo-2 benzimidazolinyl-1)-4 pipéridine, P.F. du chlorhydrate correspondant : 226-232°C, ou

de bis méta-toluoyloxy-3,4 phénylglyoxal de d'(oxo-2 benzimidazolinyl-1)-4 pipéridine, ou

de (bis méta-toluoyloxy-3,4 phényl)-1 chloro-2 éthanol-1 et d'(oxo-2 benzimidazolinyl-1)-4 pipéridine.

7) [(bis ortho-anisoyloxy-3,4 phényl)-2 hydroxy-2] éthyl-1 (oxo-2 benzimidazolinyl-1)-4 pipéridine, P.F. du chlorhydrate correspondant 162-171°C (éthanol), à partir :

de [(bis ortho-anysoyloxy-3,4 phényl)-2 oxo-2] éthyl-1 (oxo-2 benzimidazolinyl-1)-4 pipéridine,

P.F. du chlorhydrate correspondant : 180°C, ou de bis ortho-anisoyloxy-3,4 phénylglyoxal et d'(oxo-2 benzimidazolinyl-1)-4 pipéridine, ou de (bis ortho-anisoyloxy-3,4 phényl)-1 chloro-2 éthanol-1 et d'(oxo-2 benzimidazolinyl-1)-4 pipéridine.

8) [(bis méta-anisoyloxy-3,4 phényl)-2 hydroxy-2] éthyl-1 (oxo-2 benzimidazolinyl-1)-4 pipéridine, P.F. du chlorhydrate correspondant : 154-162°C(méthanol), à partir :

de [(bis méta-anisoyloxy-3,4 phényl)-2 oxo-2] éthyl-1 (oxo-2 benzimidazolinyl-1)-4 pipéridine, P.F. du chlorhydrate correspondant : 210°C, ou de bis méta-anisoyloxy-3,4 phénylglyoxal et d'(oxo -2 benzimidazolinyl-1)-4 pipéridine, ou de (bis méta-anisoyloxy-3,4 phényl)-1 chloro-2 éthanol-1 et d'(oxo-2 benzimidazolinyl-1)-4, pipéridine.

9) [(acétoxy-3 para-toluoyloxy-4 phényl)-2 hydroxy-2] éthyl-1 (oxo-2 benzimidazolinyl-1)-4 pipéridine, à partir :

d'[(acétoxy-3 para-toluoyloxy-4 phényl)-2 oxo-2] éthyl-1 (oxo-2 benzimidazolinyl-1)-4 pipéridine, ou d'acétoxy-3 para-toluoyloxy-4 phénylglyoxal et d'(oxo-2 benzimidazolinyl-1)-4 pipéridine, ou d'(acétoxy-3 para-toluoyloxy-4 phényl)-1 chloro-2 éthanol-1 et d'(oxo-2 benzimidazolinyl-1)-4 pipéridine.

10) [(bis para-toluoyloxy-3,4 phényl)-2 hydroxy-2] éthyl-1 (chloro-5 oxo-2 benzimidazolinyl-1)-4 pipéridine, P.F. du chlorhydrate correspondant : 180-190°C (éthanol), à partir :

de [(bis para-toluoyloxy-3,4 phényl)-2 oxo-2] éthyl-1 (chloro-5 oxo-2 benzimidazolinyl-1)-4 pipéridine, ou de bis para-toluoyloxy-3,4 phénylglyoxal et de (chloro-5 oxo-2 benzimidazolinyl-1) -4 pipéridine, ou

de (bis para-toluoyloxy-3,4 phényl)-1 chloro-2 éthanol-1 et de (chloro-5 oxo-2 benzimidazolinyl-1)-4 pipéridine.

11) {[bis (diméthyl-2,6 benzoyloxy)-3,4 phényl]-2 hydroxy-2} éthyl-1 (oxo-2 benzimidazolinyl-1)-4 pipéridine, P.F. du chlorhydrate correspondant 162-170°C (éthanol), à partir :

de {[bis (diméthyl-2,6 benzoyloxy)- 3,4 phényl]-2 oxo-2} éthyl-1 (oxo-2 benzimidazolinyl-1)-4 pipéridine, ou

de bis (diméthyl-2,6 benzoyloxy)-3,4 phénylglyoxal et d'(oxo-2 benzimidazolinyl-1)-4 pipéridine, ou

de [bis (diméthyl-2,6 benzoyloxy)-3,4 phényl]-1 chloro-2 éthanol-1 et d'(oxo-2 benzimidazolinyl-1) -4 pipéridine.

12) [(bis pipéronyloyloxy-3,4 phényl)-2 hydroxy-2] éthyl-1 (oxo-2 benzimidazolinyl-1)-4 pipéridine, P.F. du chlorhydrate correspondant : 196°C (éthanol/benzène), à partir :

de [(bis pipéronyloyloxy-3,4 phényl)-2 oxo-2] éthyl-1 (oxo-2 benzimidazolinyl-1)-4 pipéridine, ou

de bis pipéronyloyloxy-3,4 phénylglyoxal et d'(oxo-2 benzimidazolinyl-1)-4 pipéridine, ou

de (bis pipéronyloyloxy-3,4 phényl)-1 chloro-2 éthanol-1 et d'(oxo-2 benzimidazolinyl-1)-4 pipéridine.

13) [(formylamino-3 paratoluoyloxy-4 phényl)-2 hydroxy-2] éthyl-1 (oxo-2 benzimidazolinyl-1)-4 pipéridine, à partir :

de [(formylamino-3 paratoluoyloxy-4 phényl)-2 oxo-2] éthyl-1 (oxo-2 benzimidazolinyl-1)-4 pipéridine, ou

de formylamino-3 paratoluoyloxy-4 phénylglyoxal et d'(oxo-2 benzimidazolinyl-1)-4 pipéridine, ou

de (formylamino-3 paratoluoyloxy-4 phényl)-1 chloro-2 éthanol-1 et d'oxo-2 benzimidazolinyl-1)-4 pipéridine.

14) [(bis nicotinoyloxy-3,4 phényl)-2 hydroxy-2] éthyl-1 (oxo-2 benzimidazolinyl-1)-4 pipéridine, à partir :

de [(bis nicotinoyloxy-3,4 phényl)-2 oxo-2] éthyl-1 (oxo-2 benzimidazolinyl-1)-4 pipéridine, ou

de bis nicotinoyloxy-3,4 phénylglyoxal et d'(oxo-2 benzimidazolinyl-1)-4 pipéridine, ou

de (bis nicotinoyloxy-3,4 phényl)-1 chloro-2 éthanol-1 et d'(oxo-2 benzimidazolinyl-1)-4 pipéridine.

Revendications de brevet

1) Les dérivés de la pipéridylbenzimidazolinone de formule générale I :

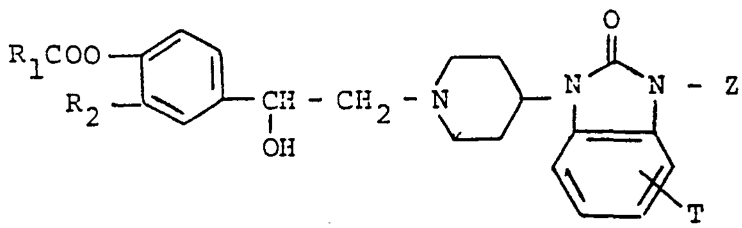

(I)

dans laquelle :

$R_1$ représente :
un radical alkyle ayant de 1 à 6 atomes de carbone, en chaîne droite ou ramifiée ;
un radical phényle non-substitué ou substitué par un ou plusieurs substituants choisis parmi les atomes d'halogène et les radicaux alkyle et alcoxy ayant chacun de 1 à 5 atomes de carbone, méthylènedioxy ou éthylènedioxy, ou
un radical thiényle, furyle ou pyridyle ;
$R_2$ représente :
un radical $R'_1$-COO- ou $R'_1$-COO-$CH_2$- dans lesquels $R'_1$ a les mêmes significations que $R_1$, $R_1$ et $R'_1$ pouvant être identiques ou différents, ou
un radical $R_3$NH- dans lequel $R_3$ représente un radical formyle, acétyle, mésyle, carbamoyle, sulfamoyle ou éthoxalyle,
Z représente un atome d'hydrogène ou un radical alkyle ou alcényle renfermant chacun jusqu'à 5 atomes de carbone en chaîne droite ou ramifiée, et
T représente un atome d'hydrogène ou d'halogène ou un radical alkyle ou alcoxy ayant chacun de 1 à 5 atomes de carbone en chaîne droite ou ramifiée.

2) Les sels des composés de la revendication 1

avec des acides appropriés.

3) Les sels selon la revendication 2 qui sont physiologiquement tolérables.

4) La [(bis para-toluoyloxy-3,4 phényl)-2 hydroxy-2] éthyl-1 (oxo-2 benzimidazolinyl-1)-4 pipéridine, et son chlorhydrate.

5) La [(bis triméthylacétoxy-3,4 phényl)-2 hydroxy-2] éthyl-1 (oxo-2 benzimidazolinyl-1)-4 pipéridine, et son chlorhydrate.

6) La [(bis para-anisoyloxy-3,4 phényl)-2 hydroxy-2] éthyl-1 (oxo-2 benzimidazolinyl-1)-4 pipéridine.

7) La [(bis benzoyloxy-3,4 phényl)-2 hydroxy-2] éthyl-1 (oxo-2 benzimidazolinyl-1)-4 pipéridine, et son chlorhydrate.

8) La [(bis ortho-anisoyloxy-3,4 phényl)-2 hydroxy-2] éthyl-1 (oxo-2 benzimidazolinyl-1)-4 pipéridine, et son chlorhydrate.

9) La [(bis méta-anisolyloxy-3,4 phényl)-2 hydroxy-2] éthyl-1 (oxo-2 benzimidazolinyl-1)-4 pipéridine, et son chlorhydrate.

10) Un procédé de préparation des composés de la revendication 1 caractérisé en ce que l'on réduit un composé de formule générale II :

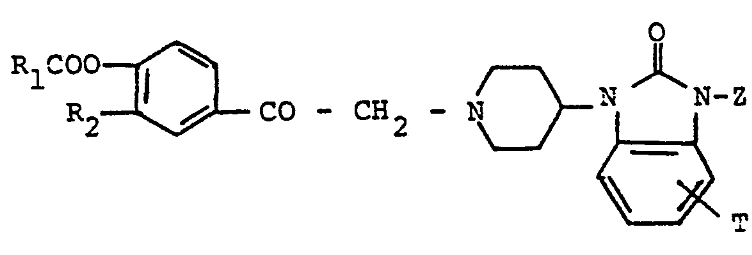

(II)

dans laquelle $R_1$, $R_2$, Z et T ont les définitions énoncées dans la revendication 1, au moyen d'un hydrure alcalin, ou d'hydrogène sous une pression comprise entre 5 et 60 bars en présence d'un catalyseur.

11) Un procédé de préparation des composés de la revendication 1, caractérisé en ce que l'on effectue une réduction alcoylante du mélange de la pipéridylbenzimidazolinone de formule générale III :

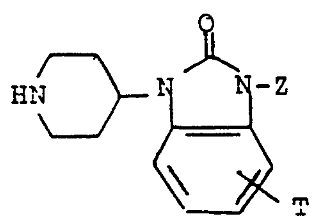

(III)

dans laquelle Z et T ont les significations énoncées dans la revendication 1,
et d'un phénylglyoxal de formule générale IV :

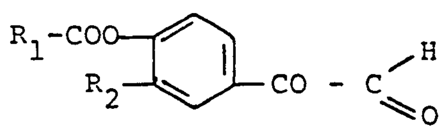

(IV)

dans laquelle $R_1$ et $R_2$ ont les significations définies dans la revendication 1.

12) Un procédé de préparation des composés de la revendication 1, caractérisé en ce que l'on fait réagir la pipéridylbenzimidazolinone de formule générale III définie dans la revendication 11, avec une halohydrine de formule générale V :

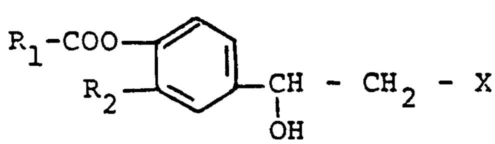

(V)

dans laquelle $R_1$ et $R_2$ ont les significations énoncées

dans la revendication 1 et X représente un atome de chlore ou de brome.

13) Les compositions pharmaceutiques contenant comme principe actif un composé selon les revendications 1 ou 3 à 9 avec les supports pharmaceutiques appropriés.

14) Les compositions pharmaceutiques selon la revendication 13 présentées sous une forme convenant notamment pour le traitement des maladies autoimmunes, allergiques et inflammatoires et pour le traitement des dyspnées notamment asthmatique et bronchique.

Office européen des brevets

# RAPPORT DE RECHERCHE EUROPEENNE

Numéro de la demande
EP 80 40 1586

## DOCUMENTS CONSIDERES COMME PERTINENTS

| Catégorie | Citation du document avec indication, en cas de besoin, des parties pertinentes | Revendication concernée |
|---|---|---|
| P | US - A - 4 200 755 (J.M. GRISAR et al.)<br>* Colonnes 1 et 2, 3 et 4 *<br>-- | 1,2,3,10,12,13,14 |
| | FR - A - 1 469 467 (JANSSEN)<br>* Revendications; page 1, colonne de droite *<br>-- | 1,2,3,10,12,13,14 |
| | FR - A - 2 213 059 (JANSSEN)<br>* Revendications *<br>-- | 1,2,3,10,12,13,14 |
| E | EP - A - 0 018 417 (KYOWA HAKKO KOGYO)<br>* Revendications; exemple 1, exemple 8 *<br>-- | 1,2,3 |
| E | EP - A - 0 008 259 (SCIENCE UNION ET CIE)<br>* Revendications *<br>---- | 1,2,3,10,11,12,13,14 |

CLASSEMENT DE LA DEMANDE (Int. Cl.³)

C 07 D 401/04
401/14
A 61 K 31/445

DOMAINES TECHNIQUES RECHERCHES (Int. Cl.³)

C 07 D 401/04
401/14
A 61 K 31/445

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

X Le présent rapport de recherche a été établi pour toutes les revendications

| Lieu de la recherche | Date d'achèvement de la recherche | Examinateur |
|---|---|---|
| La Haye | 04-03-1981 | CREMERS |

OEB Form 1503.1 06.78